(19) Europäisches Patentamt
European Patent Office
Office européen des brevets

(11) EP 0 593 227 B1

(12) EUROPEAN PATENT SPECIFICATION

(45) Date of publication and mention of the grant of the patent:
**22.03.2006 Bulletin 2006/12**

(21) Application number: **93308040.0**

(22) Date of filing: **08.10.1993**

(51) Int Cl.:
***C07D 498/18*** (2006.01) ***A61K 31/395*** (2006.01)
***A61K 31/706*** (2006.01) *C07D 498/18* (2006.01)
*C07D 311/00* (2006.01) *C07D 273/00* (2006.01)
*C07D 221/00* (2006.01)

(54) **Carbamates of rapamycin**

Carbamate von Rapamycin

Carbamates de rapamycine

(84) Designated Contracting States:
**AT BE CH DE DK ES FR GB GR IE IT LI LU NL PT SE**

(30) Priority: **13.10.1992 US 960597**
**23.04.1993 US 54655**
**24.08.1993 GB 9317596**

(43) Date of publication of application:
**20.04.1994 Bulletin 1994/16**

(60) Divisional application:
**02014573.6 / 1 266 900**

(73) Proprietor: **Wyeth**
**Madison, New Jersey 07940-0874 (US)**

(72) Inventors:
- **Kao, Wenling**
  **Chester County,**
  **Pennsylvania (US)**
- **Abou-Gharbia, Magid Abdel**
  **Glen Mills,**
  **Pennsylvania (US)**
- **Vogel, Robert Lewis**
  **Camden,**
  **New Jersey (US)**

(74) Representative: **Wileman, David Francis et al**
**C/o Wyeth Laboratories**
**Huntercombe Lane South**
**Taplow**
**Maidenhead**
**Berkshire SL6 OPH (GB)**

(56) References cited:
**WO-A-92/05179** **WO-A-92/21341**
**US-A- 4 650 803** **US-A- 5 118 677**
**US-A- 5 118 678**

- **ROCHE E.B.: 'Design of Biopharmaceutical Properties through Prodrugs and Analogs', 1988, AMERICAN PHARMACEUTICAL ASSOCIATION, ACADEMY OF PHARMACEUTICAL SCIENCES, WASHINGTON DC, US**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 593 227 B1

## Description

This invention relates to carbamates of rapamycin and a method for using them for inducing immunosuppression, and in the treatment of transplantation rejection, host vs. graft disease, autoimmune diseases, diseases of inflammation, solid tumors, fungal infections, and hyperproliferative vascular disorders.

Rapamycin is a macrocyclic triene antibiotic produced by Streptomyces hygroscopicus, which was found to have antifungal activity, particularly against Candida albicans, both in vitro and in vivo [C. Vezina et al., J. Antibiot. 28, 721 (1975); S.N. Sehgal et al., J. Antibiot. 28, 727 (1975); H. A. Baker et al., J. Antibiot. 31, 539 (1978); U.S. Patent 3,929,992; and U.S. Patent 3,993,749].

Rapamycin alone (U.S. Patent 4,885,171) or in combination with picibanil (U.S. Patent 4,401,653) has been shown to have antitumor activity. R. Martel et al. [Can. J. Physiol. Pharmacol. 55, 48 (1977)] disclosed that rapamycin is effective in the experimental allergic encephalomyelitis model, a model for multiple sclerosis; in the adjuvant arthritis model, a model for rheumatoid arthritis; and effectively inhibited the formation of IgE-like antibodies.

The immunosuppressive effects of rapamycin have been disclosed in FASEB 3, 3411 (1989). Cyclosporin A and FK-506, other macrocyclic molecules, also have been shown to be effective as immunosuppressive agents, therefore useful in preventing transplant rejection [FASEB 3, 3411 (1989); FASEB 3, 5256 (1989); R. Y. Calne et al., Lancet 1183 (1978); and U.S. Patent 5,100,899].

Rapamycin has also been shown to be useful in preventing or treating systemic lupus erythematosus [U.S. Patent 5,078,999], pulmonary inflammation [U.S. Patent 5,080,899], insulin dependent diabetes mellitus [Fifth Int. Conf. Inflamm. Res. Assoc. 121 (Abstract), (1990)], and smooth muscle cell proliferation and intimal thickening following vascular injury [Morris, R. J. Heart Lung Transplant 11 (pt. 2): 197 (1992)].

Mono- and diacylated derivatives of rapamycin (esterified at the 28 and 43 positions) have been shown to be useful as antifungal agents (U.S. Patent 4,316,885) and used to make water soluble prodrugs of rapamycin (U.S. Patent 4,650,803). Recently, the numbering convention for rapamycin has been changed; therefore according to Chemical Abstracts nomenclature, the esters described above would be at the 31- and 42- positions. U.S. Patent 5,118,678 discloses carbamates of rapamycin that are useful as immunosuppressive, anti-inflammatory, antifungal, and antitumor agents. Other derivatives of rapamycin are disclosed in WO92/05179, US 4650803 and US 5118677

DESCRIPTION OF THE INVENTION

This invention provides derivatives of rapamycin which are useful as immunosuppressive, anti-inflammatory, antifungal, antiproliferative, and antitumour agents having the structure

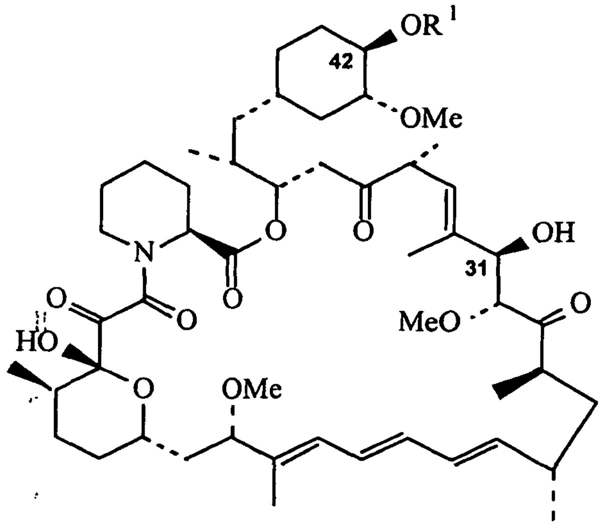

(I)

wherein $R^1$ is

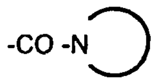

wherein

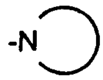

is a piperidinyl, morpholinyl, piperazinyl or pyrrolidinyl ring which may be optionally monosubstituted with a group selected from alkyl of 1-6 carbon atoms, amino, dialkylamino of 1-6 carbon atoms per alkyl group, hydroxyalkyl of 1-6 carbon atoms and alkoxyalkyl of 2-12 carbon atoms;
or a pharmaceutically acceptable salt thereof.

The pharmaceutically acceptable salts are those derived from such inorganic cations such as sodium, potassium, and the like; organic bases such as: mono-, di-, and trialkyl amines of 1-6 carbon atoms, per alkyl group and mono-, di-, and trihydroxyalkyl amines of 1-6 carbon atoms per alkyl group, and the like; and organic and inorganic acids as: acetic, lactic, citric, tartaric, succinic, maleic, malonic, gluconic, hydrochloric, hydrobromic, phosphoric, nitric, sulfuric, methanesulfonic, and similarly known acceptable acids.

It is preferred that

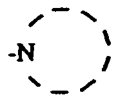

is a morpholiny or piperazinyl group that may be optionally substituted as described above.

This invention also provides processes for preparing the compounds of formula I. Accordingly this invention provides a process for preparing a compound of formula I which comprises

(a) reacting a carbonate derivative of rapamycin of formula:

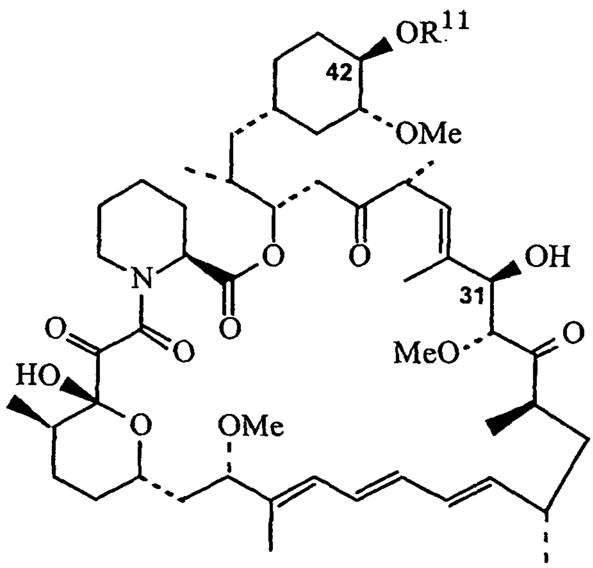

where one of $R^{11}$ is a group of formula

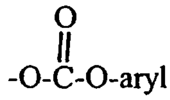

(II)

wherein aryl is an aryl group preferably substituted by electron withdrawing substituents, e.g Ar=nitrophenyl, pentachlorophenyl, with an amine of formula

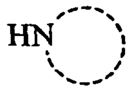

where

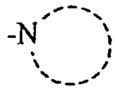

as defined above,
or
(b) carbamylating rapamycin using an haloacyl compound of formula

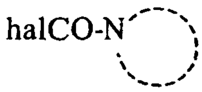

wherein

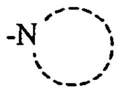

is as defined above and hal represents a halogen such as chlorine or bromine

In detail compounds of this invention carbamylated at the 42-position can be prepared by converting the 42-alcohols of rapamycin to a carbonate followed by reaction with an appropriately substituted amine to provide the desired carbamate. This scheme is illustrated below for the preparation of the compound of reference Example 2.

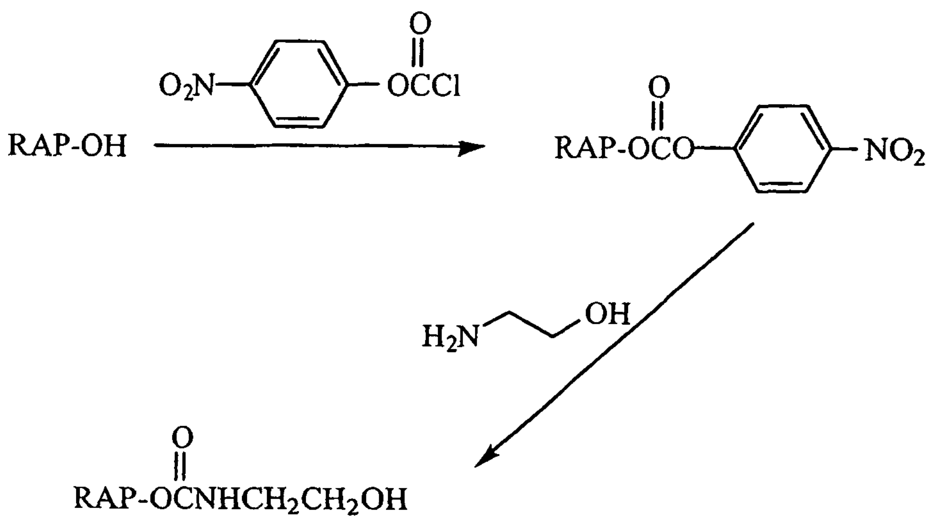

The amines used to prepare the compounds of the invention are commercially available or can be prepared by methods that are disclosed in the literature.

Immunosuppressive activity for representative compounds of this invention was evaluated in an in vitro standard pharmacological test procedure to measure lymphocyte proliferation (LAF) and in an in vivo standard pharmacological test procedure which evaluated the survival time of a pinch skin graft.

The comitogen-induced thymocyte proliferation procedure (LAP) was used as an in vitro measure of the immunosuppressive effects of representative compounds. Briefly, cells from the thymus of normal BALB/c mice are cultured for 72 hours with PHA and EL-1 and pulsed with tritiated thymidine during the last six hours. Cells are cultured with and without various concentrations of rapamycin, cyclosporin A, or test compound. Cells are harvested and incorporated radioactivity is determined. Inhibition of lymphoproliferation is assessed as percent change in counts per minute from non-drug treated controls. For each compound evaluated, rapamycin was also evaluated for the purpose of comparison. An $IC_{50}$ was obtained for each test compound as well as for rapamycin. When evaluated as a comparator for the representative compounds of this invention, rapamycin had an $IC_{50}$ ranging from 2.2 - 9.9 nM. The results obtained for the representative compounds of this invention were also expressed as a ratio compared with rapamycin. A positive ratio indicates immunosuppressive activity. A ratio of greater than 1 indicates that the test compound inhibited thymocyte proliferation to a greater extent than rapamycin. Calculation of the ratio is shown below.

$$\frac{{}^{3}\text{H-control thymus cells} - {}^{3}\text{H-rapamycin-treated thymus cells}}{{}^{3}\text{H-control thymus cells} - {}^{3}\text{H-test compound-treated cells}}$$

Representative compounds of this invention were also evaluated in an in vivo test procedure designed to determine the survival time of pinch skin graft from male BAB/c donors transplanted to male $C_3H$(H-2K) recipients. The method is adapted from Billingham R.E. and Medawar P.B., J. Exp. Biol. 28:385-402, (1951). Briefly, a pinch skin graft from the donor was grafted on the dorsum of the recipient as a allograft, and an isograft was used as control in the same region. The recipients were treated with either varying concentrations of test compounds intraperitoneally or orally. Rapamycin was used as a test control. Untreated recipients serve as rejection control. The graft was monitored daily and observations were recorded until the graft became dry and formed a blackened scab. This was considered as the rejection day. The mean graft survival time (number of days $\pm$ S.D.) of the drug treatment group was compared with the control group. The following table shows the results that were obtained. Results are expressed as the mean survival time in days. Untreated (control) pinch skin grafts are usually rejected within 6-7 days. The results shown in Table 1 are based on a dose of 4 mg/kg of test compound. A survival time of 12.0 $\pm$ 1.7 days was obtained for rapamycin at 4 mg/kg.

The following table summarizes the results of representative compounds of this invention in these two standard test procedures.

TABLE 1. EVALUATION OF IMMUNOSUPPRESSIVE ACTIVITY*

| Compound | LAF | |
|---|---|---|
| | $IC_{50}$ (nM) | (ratio) |
| Example 4 | 3.4 | 1.4 |
| Example 5 | 0.2 | 6.2 |
| Example 6 | 1.2 | 1.2 |
| Example 7 | 4.2 | 1.1 |

* Calculation of the ratio was described supra.

The results of these standard pharmacological test procedures demonstrate immunosuppressive activity both in vitro and in vivo for the compounds of this invention. Positive ratios in the LAF test procedures indicates suppression of T-cell proliferation, thereby demonstrating the immunosuppressive activity of the compounds of this invention. As transplanted pinch skin grafts are typically rejected within 6-7 days without the use of an immunosuppressive agent, the increased survival time of the skin graft when treated with the compounds of this invention further demonstrates their utility as immunosuppressive agents.

Based on the results of these standard pharmacological test procedures, the compounds are useful in the treatment or prevention of transplantation rejection such as kidney, heart, liver, lung, bone marrow, pancreas (islet cells), cornea, small bowel, and skin allografts, and heart valve xenografts; in the treatment of autoimmune diseases such as lupus, rheumatoid arthritis, diabetes mellitus, myasthenia gravis, and multiple sclerosis; and diseases of inflammation such as psoriasis, dermatitis, eczema, seborrhea, inflammatory bowel disease, pulmonary inflammation, asthma, and eye uveitis.

Because the compounds of this invention are structurally similar to rapamycin and the carbamates of rapamycin disclosed in U.S. Patent 5,118,678 and have a similar activity profile to rapamycin and the carbamates of U.S. Patent 5,118,678, the compounds of this invention also are considered to have antitumor, antifungal activities, and antiproliferative activities. The compounds of this invention therefore are also useful in treating solid tumors, fungal infections, and hyperproliferative vascular diseases such as restenosis and atherosclerosis.

It is contemplated that when the compounds of this invention are used as an immunosuppressive or antiinflammatory agent, they can be administered in conjunction with one or more other immunoregulatory agents. Such other immunoregulatory agents include, but are not limited to azathioprine, corticosteroids, such as prednisone and methylprednisolone, cyclophosphamide, rapamycin, cyclosporin A, FK-506, OKT-3, and ATG. By combining the compounds of this invention with such other drugs or agents for inducing immunosuppression or treating inflammatory conditions, lesser amounts of each of the agents are required to achieve the desired effect. The basis for such combination therapy was established by Stepkowski whose results showed that the use of a combination of rapamycin and cyclosporin A at subtherapeutic doses significantly prolonged heart allograft survival time. [Transplantation Proc. 23: 507 (1991)].

The compounds of this invention can be formulated neat or with a pharmaceutical carrier to a mammal in need thereof. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate,
magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellant.

Liquid pharmaceutical compositions which are sterile solutions or suspensions can be utilized by, for example, intramuscular, intraperitoneal or subcutaneous injection. Sterile solutions can also be administered intravenously. The compound can also be administered orally either in liquid or solid composition form.

The compounds of this invention may be administered rectally in the form of a conventional suppository. For administration by intranasal or intrabronchial inhalation or insufflation, the compounds of this invention may be formulated into an aqueous or partially aqueous solution, which can then be utilized in the form of an aerosol. The compounds of this invention may also be administered transdermally through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semipermiable membrane covering a reservoir containing the active ingredient with or without a carrier, or a matrix containing the active ingredient. Other occlusive devices are known in the literature.

In addition, the compounds of this invention may be employed as a solution, cream, or lotion by formulation with pharmaceutically acceptable vehicles containing 0.1 - 5 percent, preferably 2%, of active compound which may be administered to a fungally affected area.

The dosage requirements vary with the particular compositions employed, the route of administration, the severity of the symptoms presented and the particular subject being treated. Based on the results obtained in the standard pharmacological test procedures, projected daily dosages of active compound would be 0.1 μg/kg - 100 mg/kg, preferably between 0.001 - 25 mg/kg, and more preferably between 0.01 - 5 mg/kg. Treatment will generally be initiated with small dosages less than the optimum dose of the compound. Thereafter the dosage is increased until the optimum effect under the circumstances is reached; precise dosages for oral, parenteral, nasal, or intrabronchial administration will be determined by the administering physician based on experience with the individual subject treated. Preferably, the pharmaceutical composition is in unit dosage form, e.g. as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate number of any such compositions in package form.

The following examples illustrate the preparation of representative compounds of this invention.

**Reference Example 1**

Rapamycin 42-p-nitrophenylcarbonate

A solution of 2.0 g of rapamycin in 10 ml of dichloromethane and 2 mL of dry pyridine was cooled to -78 C under a nitrogen atmosphere. To this solution, 662 mg 4-nitrophenyl chloroformate was added; the resulting solution was stirred at room temperature under nitrogen for 20 hours. The mixture was diluted with water and extracted with dichloromethane. The dichloromethane extract was washed with water, dried over $MgSO_4$ and evaporated. The residue was chromatographed on silica gel. Elution with 33% ethyl acetate in n-hexane gave 2.07 g of rapamycin 42-p-nitrophenyl carbonate as a white foam.

**Reference Example 2**

Rapamycin 42-ester with 2-hydroxyethyl carbamic acid

A solution of 270 mg rapamycin 42-p-nitrophenyl carbonate in 8 mL dichloromethane was treated at -10 C under a nitrogen atmosphere with 61 mg ethanolamine in 0.5 mL dichloromethane. The yellow solution was stirred at 0 C under a nitrogen atmosphere for 45 minutes. The reaction mixture was diluted with 120 mL dichloromethane, washed with 1N HCl, water, dried with $MgSO_4$ and evaporated. The residue was chromatographed on silica gel. Elution with ethyl acetate/n-hexane (2/1) afforded 85 mg of the title compound as a white foam, mp 100-105 .

IR(KBr): 3430 (OH, NH), 1720 (lactone and ketone C=0)), 1640 (amide C=0), 1520, 1450, 1240, 1080, 985 and 760 $cm^{-1}$. $^1H$ NMR ($CDCl_3$, 400 MHz): 3.70 (m, 2H, $-CH_2-OH$), 3.65 (m, 2H, $-NH-CH_2$), 3.38, 3.33, 3.14 (all s, 3H, $-OCH_3$) ppm. MS (neg ion FAB): 1000 (M-), 590, 408, 297.

**Example 3**

Rapamycin 42-ester with 4-(2-hydroxyethyl)piperazine-1-carboxylic acid

A solution of 1-(2-hydroxyethyl)piperazine) (130 mg, 1.0 mmole) in 1 mL dry dichloromethane was added to a solution of 330 mg rapamycin 42-p-nitrophenyl)carbonate (0.31 mmole) in 6 mL dry dichloromethane at -8° under nitrogen and stirred at -8° for 1.5 hours. The reaction mixture was partitioned between dichloromethane and water/brine, the aqueous portion was extracted with dichloromethane, the combined organic portion was washed with brine, dried over $MgSO_4$ and evaporated to a white solid foam. Flash chromatography through silica gel using 2% methanol in dichloromethane yielded 140 mg of the title compound as a white solid, mp 112-120°C.
IR (KBr): 3450, 2950, 1725, 1650, 1460, 1250 and 995 $cm^{-1}$.
NMR ($CDCl_3$, 400 MHz): δ 3.64 (t (J = 5.2Hz), 2H, $H_d$); 3.51 (broad, 4H, $H_a$); 3.39 (s, 3H, OMe); 3.33 (s, 3H, OMe); 3.14 (s, 3H, OMe); 2.57 (t (J = 5.2 Hz), 2H, $H_c$); 2.49 (broad, 4H, $H_b$) ppm. MS (neg. ion FAB): m/z at 1069 (m-), 590.

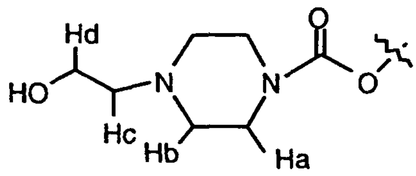

**Example 4**

Rapamycin 42-ester with 4-(3-hydroxypropyl)piperazine-1-carboxylic acid

A solution of 130 mg (0.90 mmole) of 1-(3-hydroxypropyl)piperazine in 2 mL dichloromethane was added to a solution of 320 mg (0.30 mmole) rapamycin-42-(4-nitrophenyl)carbonate in 6 mL dichloromethane under nitrogen at -5° C and allowed to warm to 20° with stirring. After 4 hours, the reaction mixture was partitioned between dichloromethane and water/brine. The organic portion was washed with brine and flash chromatographed through silica gel using methanol (2.0 to 3.0%) in dichloromethane, yielding 115 mg product as a white solid, mp 104-113° C.
IR (KBr): 3430, 2930, 1715, 1640,1450, 1240, and 985 $cm^{-1}$.
NMR (CDC13, 400 MHz): δ 3.81(t (J = 5.2 Hz), 2H, Hd); 3.49 (broad, 4H, Ha); 3.38 (s, 3H, OMe); 3.33 (s, 3H, OMe); 3.13 (s, 3H, OMe); 2.62 (t (J = 5.4 Hz), 2H, Hc); 2.48 (broad, 4H, Hb) ppm.
MS (neg ion FAB): 1083 (M-), 590.

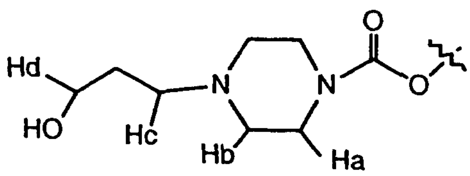

**Example 5**

Rapamycin 42-ester with morpholine-4-carboxylic acid

A solution of 95 mg (1.1 mmole) morpholine in 1 mL dry dichloromethane was added to a stirred solution of 330 mg (0.31 mmole) rapamycin-42-(4-nitrophenyl)carbonate in 6 mL dichloromethane at -5° C under nitrogen; stirring was continued 4.5 hours at -5° and 2 hours at 20°. The reaction mixture was partitioned between dichloromethane and water/brine; the organic portion was washed with brine and flash chromatographed through silica gel using methanol (1.0 to 1.6%) in dichloromethane, yielding 70 mg product as a white solid, mp 105 - 115° C.
IR (KBr): 3450, 2950, 1710, 1650, 1250, and 993 $cm^{-1}$.
NMR (CDC13, 400MHz): δ 3.64 (4H, 3-morpholine); 3.46 (t (J= 4.9 Hz), 4H, 2-morpholine); 3.37 (s, 3H, OMe); 3.32 (s, 3H, OMe); 3.12 (s, 3H, OMe) ppm.
MS (neg ion FAB): 1026 (M-), 590.

**Example 6**

Rapamycin 42-ester with 4-methylpiperazine- 1 -carboxylic acid

A solution of 95 mg (0.95 mmole) 1-methylpiperazine in 2 mL dichloromethane was added to a solution of 310 mg (0.29 mmole) rapamycin-42-(4-nitrophenyl)carbonate in 6 mL dichloromethane at 0° C under nitrogen and stirred at 0° for 2 hours and at 20° for 2 hours. The reaction mixture was partitioned between dichloromethane and water/brine. The organic portion was washed with brine and flash chromatographed through silica gel using methanol (2.0 to 3.0%) in dichloromethane, yielding 120 mg product as a white solid, mp 108 - 116° C.
IR (KBr): 3450, 2945, 1710, 1650, 1460, 1240, 1110, and 990 $cm^{-1}$.
NMR (CDCl3, 400 MHz): δ 3.50 (broad, 4H, 2-piperazine); 3.39 (s, 3H, OMe); 3.33 (s, 3H, OMe); 3.14 (s, 3H, OMe); 2.36 (broad, 4H, 3-piperazine); 2.30 (s, 3H, NMe) ppm.
MS (neg ion FAB): 1039 (M-), 590.

**Example 7**

Rapamycin 42-ester with piperazine-1-carboxylic acid

A solution of 190 mg (2.2 mmole) piperazine in 4 mL dichloromethane was added to a solution of 550 mg (0.51 mmole) rapamycin-42-(4-nitrophenyl)carbonate in 12 mL dichloromethane at 0° C under nitrogen and stirred 45 minutes. Partitioning between dichloromethane and water/brine, washing with brine and flash chromatography through silica gel using 5% methanol in dichloromethane yielded 350 mg product as a pale yellow solid, mp 120 - 131° C.
IR (KBr): 3460, 2950, 1705, 1650, 1460, 1245, and 990 $cm^{-1}$.
NMR (CDC13, 400 MHz): δ 4.8 (broad, IH, NH); 3.46 (broad, 4H, 2piperazine); 3.39 (s, 3H, OMe); 3.33 (s, 3H, OMe); 3.14 (s, 3H, OMe); 2.83 (broad, 4H, 3-piperazine) ppm.
MS (neg ion FAB): 1025 (M-), 590.

## Claims

1. A compound of the structure

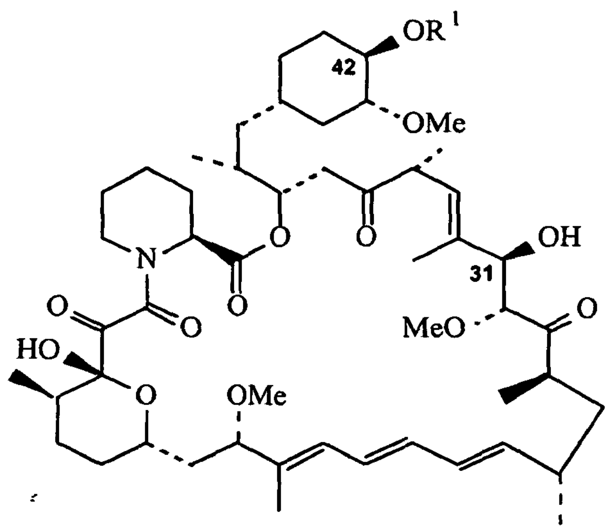

wherein $R^1$ is

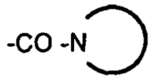

wherein

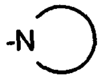

is a piperidinyl, morpholinyl, piperazinyl or pyrrolidinyl ring which may be optionally monosubstituted with a group selected from alkyl of 1-6 carbon atoms, amino, dialkylamino of 1-6 carbon atoms per alkyl group, hydroxyalkyl of 1-6 carbon atoms and alkoxyalkyl of 2-12 carbon atoms;
or a pharmaceutically acceptable salt thereof.

2. The compound according to Claim 1 wherein

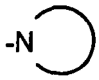

is a morpholinyl or piperazinyl group optionally substituted as defined in claim 1.

3. A compound according to Claim 1 which is one of the following:

rapamycin 42-ester with 4-(2-hydroxyethyl)piperazine-1-carboxylic acid,
rapamycin 42-ester with 4-(3-hydroxypropyl)piperazine-1-carboxylic acid,
rapamycin 42-ester with 4-methylpiperazine-1-carboxylic acid ,
rapamycin 42-ester with morpholine-4-carboxylic acid or
rapamycin 42-ester with piperazine-11-carboxylic acid,

or a pharmaceutically acceptable salt thereof.

4. A pharmaceutical composition comprising a compound as claimed in any one of Claims 1 to 3.

5. A process for preparing a compound as claimed in Claim 1 which comprises

(a) reacting a carbonate derivative of rapamycin of formula:

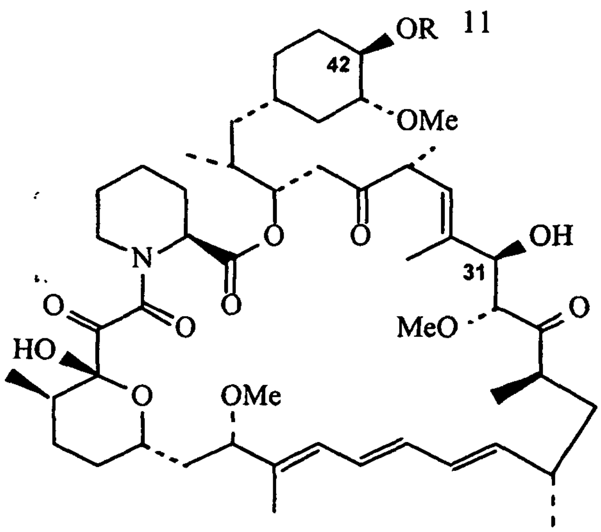

where $R^{11}$ and group of formula

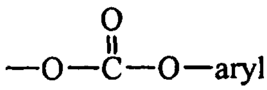

(II)

wherein aryl is an aryl group preferably substituted by electron withdrawing substituents, or $R^{11}$ with an amine of formula

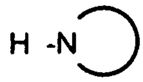

where

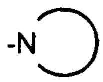

is as defined in Claim 1,
or
(b) carbamylating rapamycin, using an haloacyl compound of formula

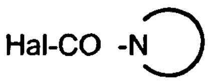

wherein

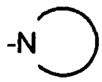

is as defined in Claim 1 and Hal represents a halogen, and if desired converting to a pharmaceutically acceptable salt thereof.

## Revendications

**1.** Composé de structure:

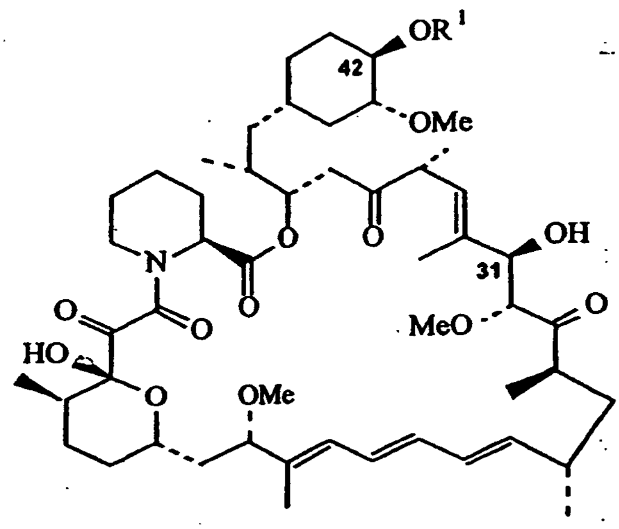

dans laquelle $R^1$ est

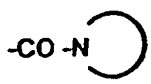

dans laquelle

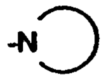

est un noyau pipéridinyle, morpholinyle, pipérazinyle ou pyrrolidinyle qui peut être facultativement monosubstitué par un groupement sélectionné parmi les groupes alkyle de 1 à 6 atomes de carbone, amino, dialkylamino de 1 à 6 atomes de carbone par groupe alkyle, hydroxyalkyle de 1 à 6 atomes de carbone et alcoxyalkyle de 2 à 12 atomes de carbone ;
ou un sel pharmaceutiquement acceptable de ces composés.

**2.** Composé selon la revendication 1, dans lequel

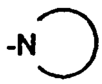

est un groupe morpholinyle ou pipérazinyle facultativement substitué comme défini dans la revendication 1.

**3.** Composé selon la revendication 1, qui est l'un des suivants:

rapamycine 42-ester de l'acide 4-(2-hydroxyéthyl)pipérazine-1-carboxylique,
rapamycine 42-ester de l'acide 4-(3-hydroxypropyl)pipérazine-1-carboxylique,
rapamycine 42-ester de l'acide 4-méthylpipérazine-1-carboxylique,
rapamycine 42-ester de l'acide morpholine-4-carboxylique ou
rapamycine 42-ester de l'acide pipérazine-1-carboxylique

ou un sel pharmaceutiquement acceptable de ces composés.

**4.** Composition pharmaceutique comprenant un composé selon l'une quelconque des revendications 1 à 3.

**5.** Procédé de préparation d'un composé selon la revendication 1, qui consiste à:

(a) faire réagir un dérivé carbonate de la rapamycine de formule:

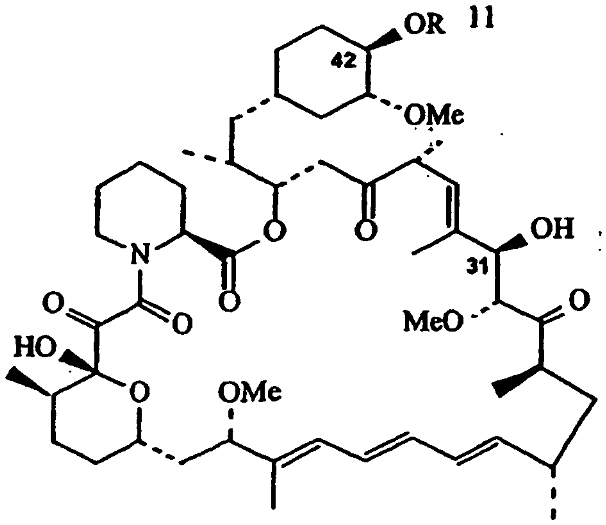

où $R^{11}$ est un groupe de formule

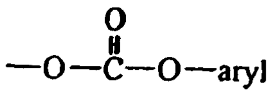

(II)

dans lequel aryle est un groupe aryle substitué de préférence par des substituants extracteurs d'électrons, ou $R^{11}$ avec une amine de formule

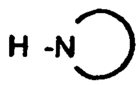

où

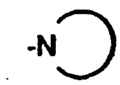

est tel que défini dans la revendication 1,
ou
(b) carbamyler la rapamycine, au moyen d'un composé haloacyle de formule

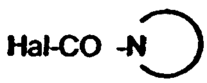

dans laquelle

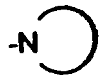

est tel que défini dans la revendication 1 et Hal représente un halogène, et si on le souhaite en le convertissant en un sel phamaceutiquement acceptable.

## Patentansprüche

1. Verbindung mit der Struktur

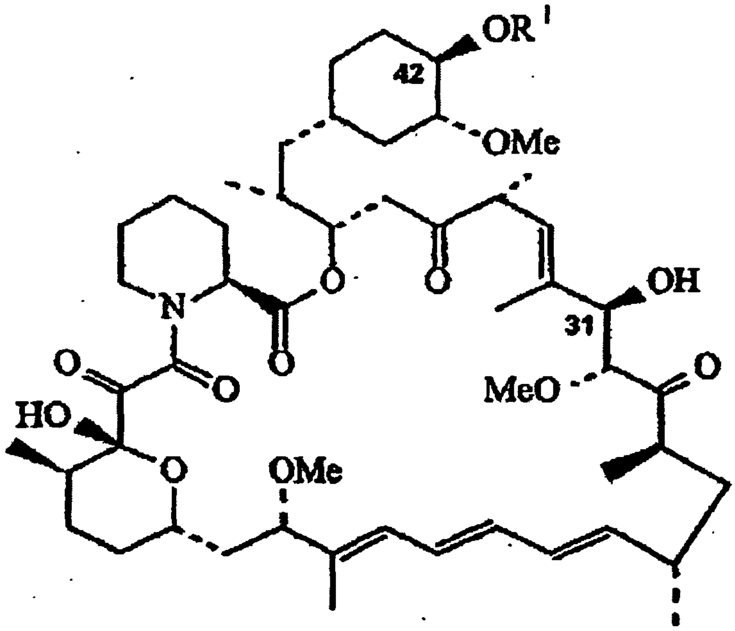

worin $R^1$ für

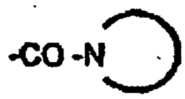

steht, worin

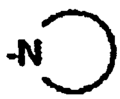

für einen Piperidinyl-, Morpholinyl-, Piperazinyl- oder Pyrrolidinylring steht, welcher gegebenenfalls monosubstituiert sein kann mit einer Gruppe, ausgewählt aus Alkyl mit 1-6 Kohlenstoffatomen, Amino, Dialkylamino mit 1-6 Kohlenstoffatomen pro Alkylgruppe, Hydroxyalkyl mit 1-6 Kohlenstoffatomen und Alkoxyalkyl mit 2-12 Kohlenstoffatomen; oder ein pharmazeutisch annehmbares Salz davon.

2. Verbindung gemäß Anspruch 1, worin

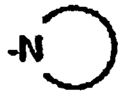

für eine Morpholinyl- oder Piperazinylgruppe steht, gegebenenfalls substituiert wie in Anspruch 1 definiert.

3. Verbindung gemäß Anspruch 1, welche eine der folgenden ist:

Rapamycin-42-ester mit 4-(2-Hydroxyethyl)piperazin-1-carbonsäure
Rapamycin-42-ester mit 4-(3-Hydroxypropyl)piperazin-1-carbonsäure,
Rapamycin-42-ester mit 4-Methylpiperazin-1-carbonsäure,
Rapamycin-42-ester mit Morpholin-4-carbonsäure oder
Rapamycin-42-ester mit Piperazin-1-carbonsäure

oder ein pharmazeutisch annehmbares Salz davon.

4. Pharmazeutische Zusammensetzung, welche eine Verbindung umfasst, wie in einem der Ansprüche 1 bis 3 beansprucht.

5. Verfahren zum Herstellen einer Verbindung wie in Anspruch 1 beansprucht, welches umfasst:

(a) Umsetzen eines Carbonatderivats von Rapamycin mit der Formel:

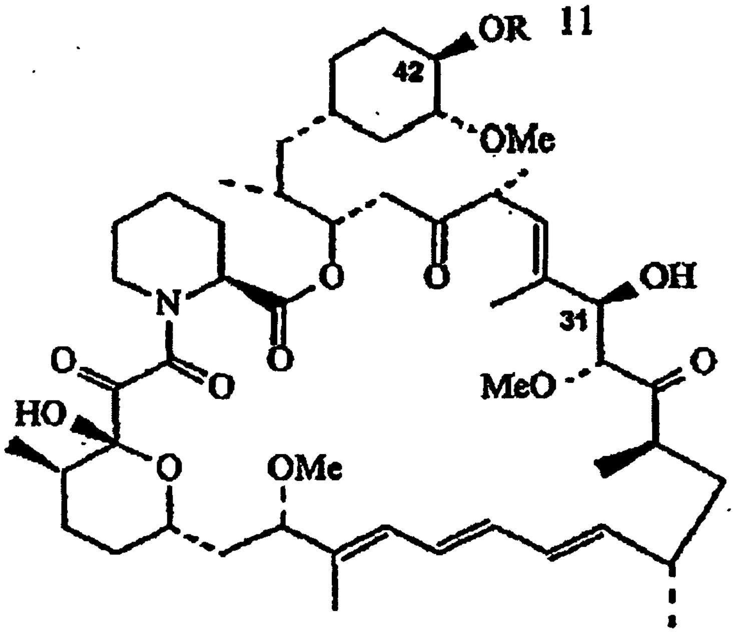

worin $R^{11}$ eine Gruppe der Formel

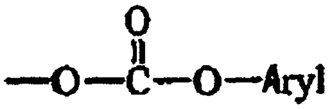

(II)

darstellt, worin Aryl eine Arylgruppe darstellt, vorzugsweise substituiert durch Elektronen-abziehende Substi-

tuenten, oder $R^{11}$ mit einem Amin der Formel

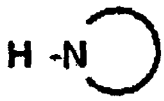

worin

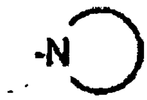

wie in Anspruch 1 definiert ist,
oder
(b) Carbamoylieren von Rapamycin unter Verwendung einer Halogenacylverbindung der Formel

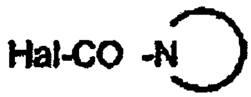

worin

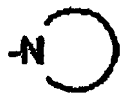

wie in Anspruch 1 definiert ist und Hal ein Halogen darstellt, und falls gewünscht, Umwandeln in ein pharmazeutisch annehmbares Salz davon.